# EUROPEAN PATENT APPLICATION

(11) **EP 4 835 014 A1**
(43) Date of publication of application: **04.11.2026**
(21) Application number: 24909732.0
(22) Date of filing: 30.07.2024
(51) Int. Cl.: C09D 5/16, A61L 31/14

(54) **NEW ANTIFOULING DEVELOPING COATING MATERIAL, AND PREPARATION METHOD THEREFOR AND USE THEREOF**

(30) Priority: 27.12.2023 CN 202311820422; 28.06.2024 CN 202410861298
(71) Applicant: Suzhou Silver Mars New Materials Technology Co., Ltd, Suzhou, Jiangsu 215000 (CN)
(72) Inventor: XUE, Bin, Suzhou, Jiangsu 215000 (CN); YANG, Xin, Suzhou, Jiangsu 215000 (CN); ZHANG, Qiuhong, Suzhou, Jiangsu 215000 (CN); LI, Li, Suzhou, Jiangsu 215000 (CN); QIU, Zhenye, Suzhou, Jiangsu 215000 (CN)
(74) Representative: HGF
(86) International application number: PCT/CN2024/108527
(87) International publication number: WO 2025/138778

(57) **Abstract**

A new antifouling developing coating material, and a preparation method therefor and the use thereof. In the antifouling developing coating material, barium ions are combined with the sulfonate anions, and the barium ions are introduced into the structure of a modified choline phosphate polymer, which is modified with siloxane and sulfonate anions, thereby allowing the material to have combined antifouling and developing functions; therefore, the stability of the material is ensured, and the material has wide application prospects in the field of medical imaging.

## Description

### TECHNICAL FIELD

The present application relates to the technical field of medical materials, for example, a novel antifouling radiopaque coating material, a preparation method therefor and the use thereof.

### BACKGROUND

Medical radiopaque coatings play a critical role in medical imaging. Advancing medical imaging requires better radiopaque efficacy from the radiopaque coatings. Radiopaque coatings also must possess excellent antifouling capabilities and can effectively resist the attachment of bacteria and proteins to prevent infections, while maintaining a clean and transparent surface to ensure clear and accurate imaging.

Medical antifouling radiopaque coatings develop forward by improving the conventional radiopaque coatings. Conventional radiopaque coatings provide radiopaque functionality, but their antifouling capabilities are limited. With the increasing use of medical devices, there is a growing need for coatings having improved antifouling properties. The antifouling mechanism primarily involves the hydrophilicity and lipophilicity of material surfaces. Hydrophilic surfaces have large contact angles with water, preventing moisture and impurities from adhering. Hydrophobic surfaces resist the adhesion of organic matter or oils, reducing the adsorption of biomolecules. In recent years, significant progress has been made in the research of medical antifouling coatings. Researchers have modified surface properties to develop coatings with superior antifouling performance. For example, superhydrophobic polymer materials are introduced to create superhydrophobic effects on the surfaces, enabling efficient self-cleaning and antifouling. Meanwhile, biomimetic designs have been employed to create special surface structures, such as convex and concave micro- or nano-structures and honeycomb-like structure, reducing contact areas and minimizing contaminant adhesion. Bioactive molecules embedded in the coatings form "antimicrobial zones" that inhibit microbial proliferation, further enhancing antifouling effectiveness.

The radiopaque performance of medical antifouling radiopaque coatings critically impacts the clarity of medical images and the accuracy of diagnoses. These radiopaque coatings must be sensitive to X-rays to ensure effective imaging results under X-ray exposure. Radiopaque technology has been widely used in medical imaging, where clear images are formed based on the imaging efficiency of photosensitive materials. X-ray contrast is generally achieved through the absorption and scattering of X-rays by radiopaque materials. The radiopaque coatings play a key role in this process by absorbing and scattering X-rays to form images. Research on medical radiopaque coatings has evolved alongside advancements in medical imaging technology. Researchers have improved the radiopaque agents and chemical compositions of radiopaque coatings to enhance radiopaque efficiency and image quality.

However, the mutual interference between imaging agents and antifouling agents may reduce imaging efficiency; therefore, the effective combination of imaging and antifouling functions remains a challenging issue that needs to be addressed.

### SUMMARY

The following is a summary of subject matters described in detail herein. The summary is not intended to limit the scope of the claims.

The present application provides a novel antifouling radiopaque coating material, a preparation method therefor and the use thereof.

In an aspect, the present application provides a novel antifouling radiopaque coating material. The antifouling radiopaque coating material has a structure represented by Formula I: 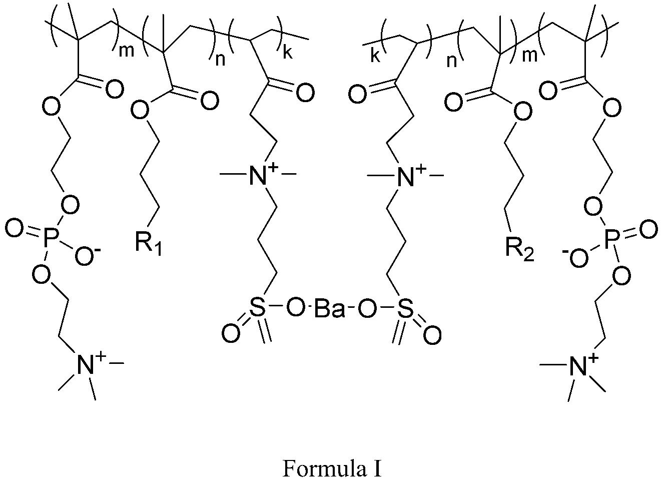 where m = 20, n = 10, k = 3, R₁ and R₂ each are a siloxane-containing group, and R₁ and R₂ are the same or different.

In the antifouling radiopaque coating material of the present application, through the combination of barium ions with sulfonate groups, the barium ions are introduced into the structure of a phosphorylcholine polymer modified with siloxane and sulfonate groups, thereby enabling the obtained material to combine antifouling properties and radiopaque functions, ensuring the stability of the material, and enabling the stability of the antifouling and radiopaque performance. Therefore, the material exhibits outstanding performance in both the antifouling capability and the radiopaque efficacy, thereby providing the material with broad application prospects in the field of medical imaging and offering strong support for the safety of medical devices and the accuracy of medical imaging.

In some optional embodiments, m:n = 1:1 - 4:1, for example, 1:1, 2:1, 3:1 or 4:1. In the present application, when the ratio of m:n is within the range of 1:1 to 4:1, by adjusting the molar ratio of the modified phosphorylcholine polymer to the barium salt, the polymer material satisfying the requirements for radiopaque and antifouling properties can be obtained.

In some optional embodiments, R₁ and R₂ are independently selected from tris(trimethylsiloxy)silane or trimethoxysilane, the structures of which are as follows:

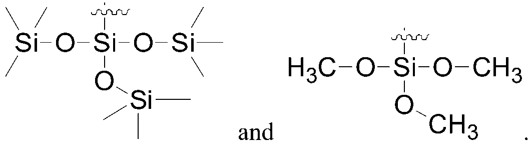

In the present application, m, n, and k represent the numbers of structural units, where R₁ in n structural units may be the same or different, and R₂ in n structural units may be the same or different.

In another aspect, the present application provides a method for preparing the novel antifouling radiopaque coating material described above. The preparation method includes the following steps.
(1) A phosphorylcholine polymer modified with siloxane and sulfonate groups is prepared.
(2) A barium salt is added to a solution containing the phosphorylcholine polymer modified with siloxane and sulfonate groups, and the mixture reacts to obtain the novel antifouling radiopaque coating material.

Optionally, step (1) specifically includes the following step: 2-methacryloyloxyethyl phosphorylcholine reacts with a siloxane compound and (2-(methacryloyloxy)ethyl)dimethyl-(3-sulfopropyl)ammonium hydroxide to obtain the phosphorylcholine polymer modified with siloxane and sulfonate groups.

Optionally, the siloxane compound is methacryloxypropyltris(trimethylsiloxy)silane, methacryloxypropyltris(trimethoxy)silane, or vinyltrimethoxysilane.

Optionally, the molar ratio of 2-methacryloyloxyethyl phosphorylcholine to the siloxane compound is 1:1 - 8:1, for example, 1:1, 1.5:1, 2:1, 2.5:1, 3:1, 3.5:1, 4:1, 4.5:1, 5:1, 5.5:1, 6:1, 6.5:1, 7:1, 7.5:1 or 8:1.

Optionally, the reaction between 2-methacryloyloxyethyl phosphorylcholine and the siloxane compound is carried out in the presence of 4-cyano-4-(thiobenzoylthio)pentanoic acid.

Optionally, the molar ratio of 2-methacryloyloxyethyl phosphorylcholine to 4-cyano-4-(thiobenzoylthio)pentanoic acid is 1:1 - 5:1, for example, 1:1, 1.5:1, 2:1, 2.5:1, 3:1, 3.5:1, 4:1, 4.5:1 or 5:1.

Optionally, the reaction between 2-methacryloyloxyethyl phosphorylcholine and the siloxane compound is carried out in the presence of an initiator.

Optionally, the initiator is selected from azobisisobutyronitrile.

Optionally, the molar ratio of 2-methacryloyloxyethyl phosphorylcholine to the initiator is 1:1 - 5:1, for example, 1:1, 1.5:1, 2:1, 2.5:1, 3:1, 3.5:1, 4:1, 4.5:1 or 5:1.

Optionally, the molar ratio of 2-methacryloyloxyethyl phosphorylcholine to (2-(methacryloyloxy)ethyl)dimethyl-(3-sulfopropyl)ammonium hydroxide is 2:1 - 8:1, for example, 2:1, 2.5:1, 3:1, 3.5:1, 4:1, 4.5:1, 5:1, 5.5:1, 6:1, 6.5:1, 7:1, 7.5:1 or 8:1.

Optionally, the reaction in step (1) is carried out at a temperature of 60 °C - 70 °C (for example, 60 °C, 63 °C, 65 °C, 68 °C or 70 °C) for 16 - 64 h (for example, 16 h, 18 h, 20 h, 24 h, 28 h, 30 h, 36 h, 40 h, 42 h, 48 h, 50 h, 55 h, 58 h, 60 h or 64 h).

Optionally, the solvent for the reaction in step (1) is one or a mixture of at least two of *n*-propanol, methanol, acetone, tetrahydrofuran, *n*-butanol, or isopropanol.

Optionally, the reaction in step (1) is carried out under nitrogen protection.

Optionally, step (1) includes the following more specific operations: 2-methacryloyloxyethyl phosphorylcholine, 4-cyano-4-(thiobenzoylthio)pentanoic acid, and azobisisobutyronitrile are dissolved in *n*-propanol; and under nitrogen protection, the mixture is heated to 60 °C - 70 °C and stirred to react for 16 - 32 h, a siloxane compound and (2-(methacryloyloxy)ethyl)dimethyl-(3-sulfopropyl)ammonium hydroxide are added, and the reaction is continued for another 16 - 32 h to obtain the phosphorylcholine polymer modified with siloxane and sulfonate groups.

Optionally, after the reaction in step (1) is completed, the preparation method further includes a post-treatment step, where the post-treatment step includes: the obtained reaction solution is precipitated in diethyl ether, filtered, and dried.

In the present application, the barium salt in step (2) is selected from one or a mixture of at least two of barium chloride, barium sulfate, barium nitrate, or barium carbonate.

Optionally, the barium salt is barium chloride.

Optionally, the molar ratio of the phosphorylcholine polymer modified with siloxane and sulfonate groups to the addition amount of the barium salt in step (2) ranges from 1:1 to 4:1, for example, 1:1, 1.5:1, 1.8:1, 2:1, 2.3:1, 2.5:1, 2.8:1, 3:1, 3.3:1, 3.5:1, 3.8:1 or 4:1, and optionally, is 1:1.

Optionally, the solvent in the solution in step (2) is a mixed solvent of methanol and water.

Optionally, the reaction in step (2) is carried out at room temperature for 6 - 12 h (for example, 6 h, 7 h, 8 h, 9 h, 10 h, 11 h or 12 h).

In another aspect, the present application provides the use of the novel antifouling radiopaque coating material described above in coating a substrate surface.

Optionally, the substrate surface includes a silicon-based surface, a glass substrate surface, a metal substrate surface, or a polymer substrate surface.

The material of the present application can be applied to a variety of substrate surfaces, including glass substrates, metal substrates, and various plastic and polymer substrates (silicone rubber, polyethylene, polypropylene, polyvinyl chloride, polystyrene or polyurethane).

The novel antifouling radiopaque coating material of the present application has broad applicability. The material enables covalent bonding to any silicon-based substrate (including various silicon-based surfaces such as glass, silicone sheets, and marble) through the bonding to hydroxyl groups on the substrate surface via silicon-oxygen bonds, and the material can be bound to other substrate surfaces through hydrophobic interactions or van der Waals forces, thereby making the material widely applicable to different clinical and laboratory scenarios and diverse needs of users.

In another aspect, the present application provides an antifouling radiopaque coating, where the raw material for preparing the antifouling radiopaque coating includes the novel antifouling radiopaque coating material described above.

In another aspect, the present application provides the use of the novel antifouling radiopaque coating material described above in a medical device, a medical material, an optical lens or industrial printing.

Compared with the related art, the present application has the following beneficial effects.

First, the novel antifouling radiopaque coating material of the present application has been successfully applied to medical devices, forming an antifouling coating with excellent antifouling performance. Compared with the conventional radiopaque coating, the novel antifouling radiopaque coating material achieves a significant improvement in antifouling performance. The material contains a modified phosphorylcholine polymer as a unique antifouling component, which can effectively resist the adhesion of bacteria and proteins and prevent the occurrence of bacterial infections and cross-infections. This characteristic is crucial for medical safety, ensuring that medical devices remain hygienic and safe during use. The material is applicable to various medical scenarios and is of particular importance for equipment requiring high hygiene standards, such as operating rooms and medical catheters.

Second, the novel antifouling radiopaque coating material of the present application exhibits an excellent radiopaque performance. Compared with conventional methods, the surface of the material is in the form of an elastomer coating that is insoluble in the aqueous phase, and the material does not release from the substrate, ensuring the stability and durability of the radiopaque performance. Therefore, the material eliminates potential side effects caused by the radiopaque coating and improves the safety of medical radiopaque coatings, which has positive contribution to the health and medical experience of patients.

In addition, the novel antifouling radiopaque coating material of the present application has broad applicability. The material enables covalent bonding to any silicon-based substrate (including various silicon-based surfaces such as glass, silicone sheets, and marble) through the bonding to hydroxyl groups on the substrate surface via silicon-oxygen bonds, and the material can be bound to other substrate surfaces through hydrophobic interactions or van der Waals forces, thereby making the material widely applicable to different clinical and laboratory scenarios and diverse needs of users. The preparation method of the material is simpler and lower in cost compared with the conventional coating, thereby providing a feasible approach for the large-scale application of radiopaque coatings.

Other aspects can be understood after the drawings and the detailed description are read and understood.

### BRIEF DESCRIPTION OF DRAWINGS

The drawings, which are intended to facilitate a further understanding of the technical solutions described herein and constitute part of the specification, together with the embodiments of the present application, are used to explain the technical solutions herein and shall not be construed as limiting the technical solutions herein.
FIG. 1 shows the nuclear magnetic resonance (NMR) spectrum of a modified phosphorylcholine polymer obtained by reacting a mixture of methacryloxypropyltris(trimethylsiloxy)silane and (2-(methacryloyloxy)ethyl)dimethyl-(3-sulfopropyl)ammonium hydroxide.
FIG. 2 shows the NMR comparison between a polymer obtained in Example 1 and a modified phosphorylcholine polymer obtained by reacting a mixture of *n*-hexyltriethoxysilane and (2-(methacryloyloxy)ethyl)dimethyl-(3-sulfopropyl)ammonium hydroxide.
FIG. 3 shows the microscopic morphology of the novel antifouling radiopaque coating material of the present application coated on different substrates, where A is a photograph of the coating on a glass slide, B is a photograph of the coating on a silicone sheet, C is a photograph of the coating on a polyurethane sheet, and D is a photograph of the coating on the tip of a stainless steel syringe needle; in each photograph, the arrow points to the edge of the coating.
FIG. 4A is a fluorescence microscopy photograph showing results of the protein-adhesion resistance test of the novel antifouling radiopaque coating material of the present application.
FIG. 4B shows the results of the fluorescence quantitative analysis of the total amount of protein molecules adsorbed on the novel antifouling radiopaque coating material of the present application.
FIGS. 5A and 5B show the results of the bacterial-adhesion resistance test of the novel antifouling radiopaque coating material of the present application, where FIG. 5A is a photograph of the bacterial-adhesion resistance test results, and FIG. 5B is a graph showing the quantitative analysis results of adsorbed bacterial colonies.
FIGS. 6A and 6B show the results of the platelet-adhesion resistance test of the novel antifouling radiopaque coating material of the present application, where FIG. 6A is a photograph of the platelet-adhesion resistance results, and FIG. 6B is a graph showing the quantitative analysis results of the quantity of adsorbed platelets.
FIGS. 7A and 7B show X-ray imaging results of the novel antifouling radiopaque coating material of the present application after being coated on substrates, where FIG. 7A shows X-ray imaging photographs using different radiopaque methods, and FIG. 7B is a graph showing the normalized radiopaque intensity analysis.

### DETAILED DESCRIPTION

Technical solutions of the present application are further described below through specific examples. It is to be understood by those skilled in the art that the examples are only intended to facilitate understanding of the present application and are not to be construed as limiting the present application.

### Example 1

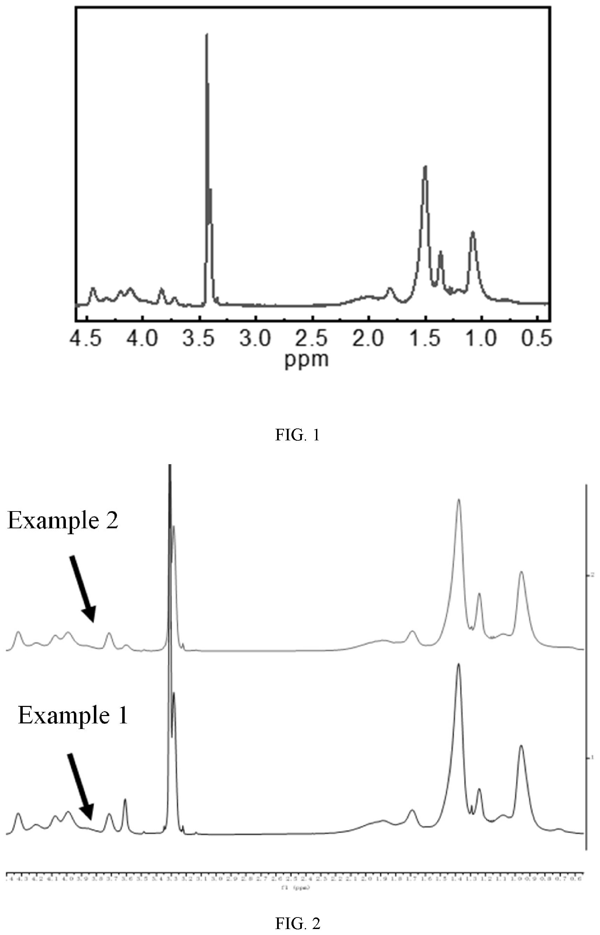

This example provides a novel antifouling radiopaque coating material. The novel antifouling radiopaque coating material was prepared by a method including the following steps. 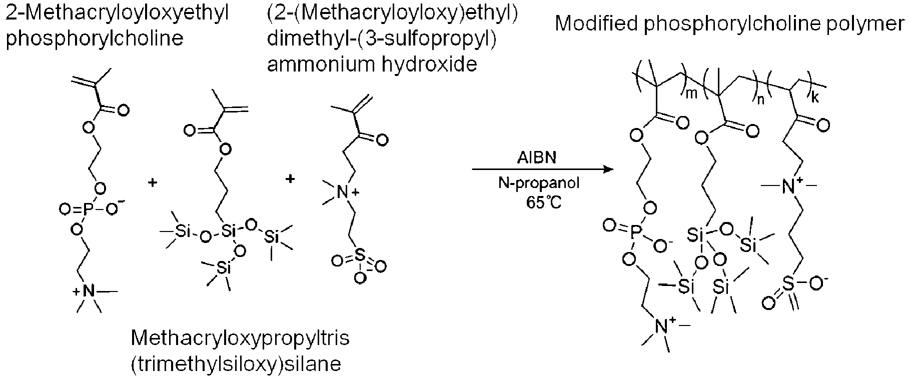
1) 0.75 mol of 2-methacryloyloxyethyl phosphorylcholine, 0.4 mol of 4-cyano-4-(thiobenzoylthio)pentanoic acid, and 0.4 mol of azobisisobutyronitrile were dissolved in n-propanol. Under nitrogen protection, the resulting mixture was slowly heated to 65 °C and stirred to react for 24 h. Then, a mixture of 0.125 mol of methacryloxypropyltris(trimethylsiloxy)silane and 0.075 mol of (2-(methacryloyloxy)ethyl)dimethyl-(3-sulfopropyl)ammonium hydroxide was added, and the reaction was continued for another 24 h. The resulting n-propanol solution was precipitated in diethyl ether, filtered, and dried to obtain a modified phosphorylcholine polymer. The reaction scheme is shown above, and the NMR results of the polymer are shown in FIG. 1.
2) Barium chloride was dissolved in double-distilled water by a concentration of 200 mg/mL and then mixed with 600 mg/mL of the modified phosphorylcholine polymer in a methanol/water solvent to a total concentration of 400 mg/mL. The mixture was stirred thoroughly at room temperature for 6 h to allow the barium ions to bind with the sulfonate groups of the modified phosphorylcholine polymer, thereby forming an antifouling radiopaque polymer capable of being coated onto any silicon-based substrate, that is, the antifouling radiopaque coating material. The molecular structure of the antifouling radiopaque coating material is as follows: 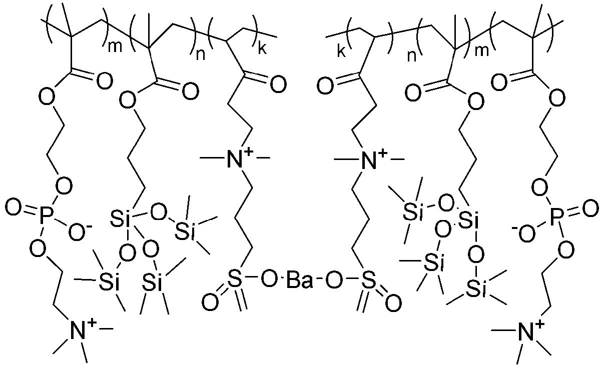 where m = 20, n = 10, and k = 3.

### Example 2

1) 0.75 mol of 2-methacryloyloxyethyl phosphorylcholine, 0.4 mol of 4-cyano-4-(thiobenzoylthio)pentanoic acid, and 0.4 mol of azobisisobutyronitrile were dissolved in n-propanol. Under nitrogen protection, the resulting mixture was slowly heated to 65 °C and stirred to react for 24 h. Then, a mixture of 0.125 mol of vinyltrimethoxysilane and 0.075 mol of (2-(methacryloyloxy)ethyl)dimethyl-(3-sulfopropyl)ammonium hydroxide was added, and the reaction was continued for another 24 h. The resulting n-propanol solution was precipitated in diethyl ether, filtered, and dried to obtain a modified phosphorylcholine polymer. The NMR results of the polymer were compared with the NMR results of the polymer obtained in Example 1, as shown in FIG. 2.
2) Barium chloride was dissolved in double-distilled water by a concentration of 200 mg/mL and then mixed with 600 mg/mL of the modified phosphorylcholine polymer in a methanol/water solvent to a total concentration of 400 mg/mL. The mixture was stirred thoroughly at room temperature for 6 h to allow the barium ions to bind with the sulfonate groups of the modified phosphorylcholine polymer, thereby forming an antifouling radiopaque polymer capable of being coated onto any silicon-based substrate, that is, the antifouling radiopaque coating material. The molecular structure of the antifouling radiopaque coating material is as follows: 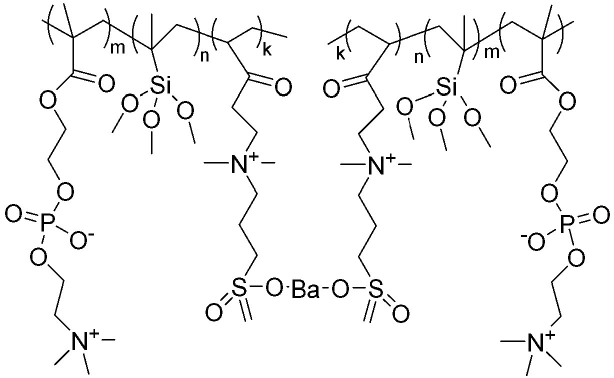 where m = 20, n = 10, and k = 3.

### Example 3

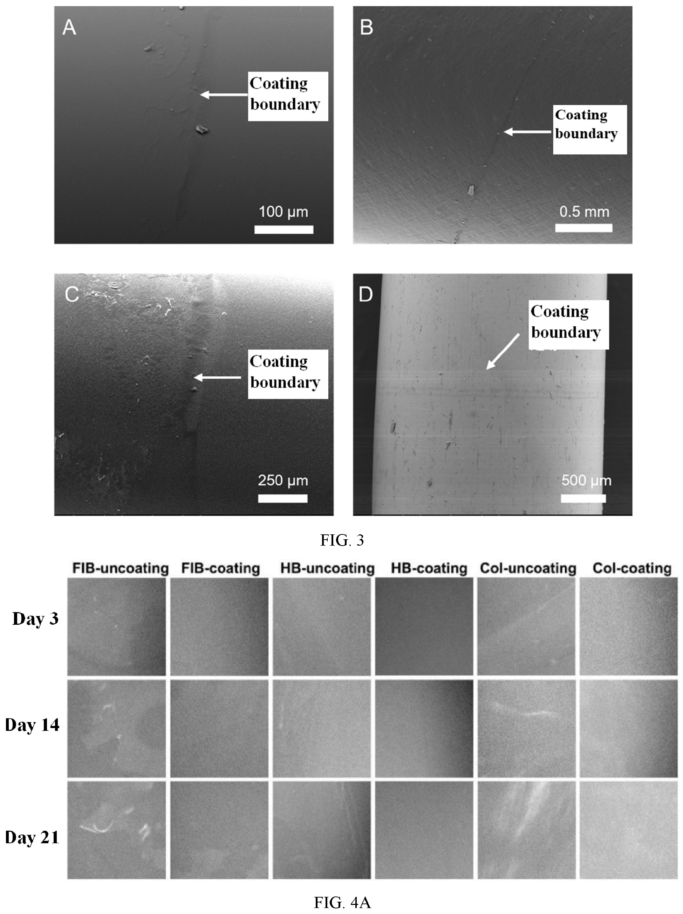

The antifouling radiopaque coating material of Example 1 was coated on a glass slide, a silicone sheet, a polyurethane sheet, and the tip of a stainless steel syringe needle to form antifouling radiopaque coatings. Morphological observation was performed using a scanning electron microscope (S-3400N). The results are shown in FIG. 3, where A is the SEM image of the coating on the glass slide, B is the SEM image of the coating on the silicone sheet, C is the SEM image of the coating on the polyurethane sheet, and D is the SEM image of the coating on the tip of the stainless steel syringe needle. As can be seen, the antifouling radiopaque polymer-coated areas had smooth surface overall, there was a clear boundary between the coated areas and the uncoated areas, and the coated areas showed no obvious defects or cracks, demonstrating the stability of the antifouling radiopaque material of the present application when coated on different substrates.

### Example 4

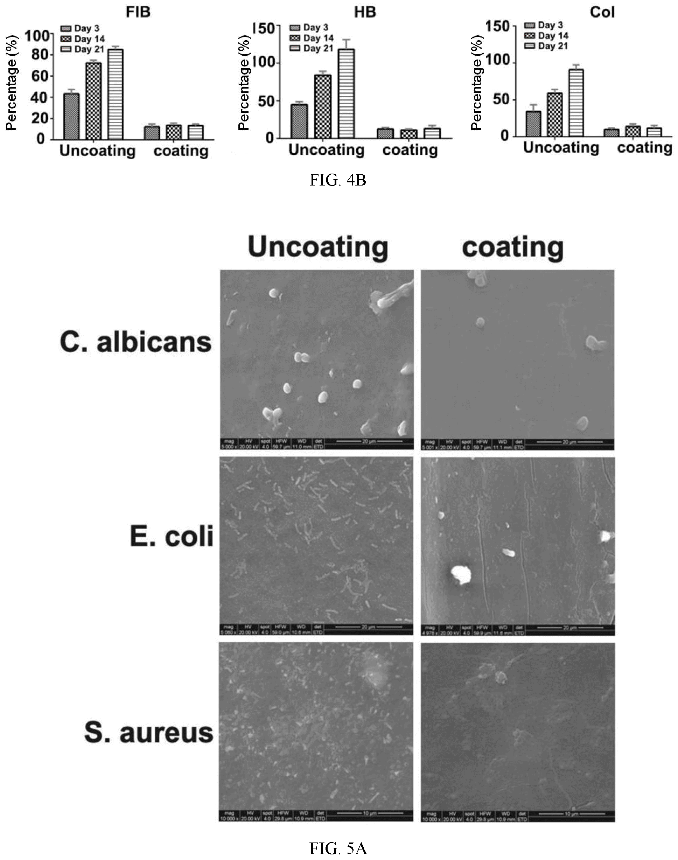

The antifouling radiopaque coating material of Example 1 was tested for anti-protein adhesion. Regarding protein adsorption resistance, the adsorption capacities of fibrin (FIB), serum protein (HB), and collagen (Col) on the antifouling radiopaque coating were measured and characterized. The protein molecules were pre-labeled with fluorescent molecules, and an adsorption experiment was performed for 21 days. On days 3, 14, and 21, the adsorption of various molecules on the coating was observed using a fluorescence microscope (Nikon Eclipse TE/Ti). The control groups were blank controls without the antifouling radiopaque coating. The results are shown in FIGS. 4A and 4B (in the figures, days 3, 14, and 21 are labeled as Day 3, Day 14, and Day 21, respectively; "Uncoating" indicates no coating, and "Coating" indicates coating; these have the same meanings in the figures below).

As shown in FIG. 4A, compared with the blank controls, the coating exhibited excellent protein adsorption resistance. Serum proteins were barely adsorbed to the coating, and the adsorption of fibrin on the antifouling radiopaque coating was extremely low, whereas the blank control groups were covered with adsorbed protein molecules. Furthermore, the fluorescence quantitative analysis was performed to obtain the total amount of adsorbed molecules. As shown in FIG. 4B, compared with the blank control groups, the total amount of protein adsorbed on the antifouling radiopaque coating was significantly reduced, demonstrating the high protein-adsorption resistance of the antifouling radiopaque coating.

### Example 5

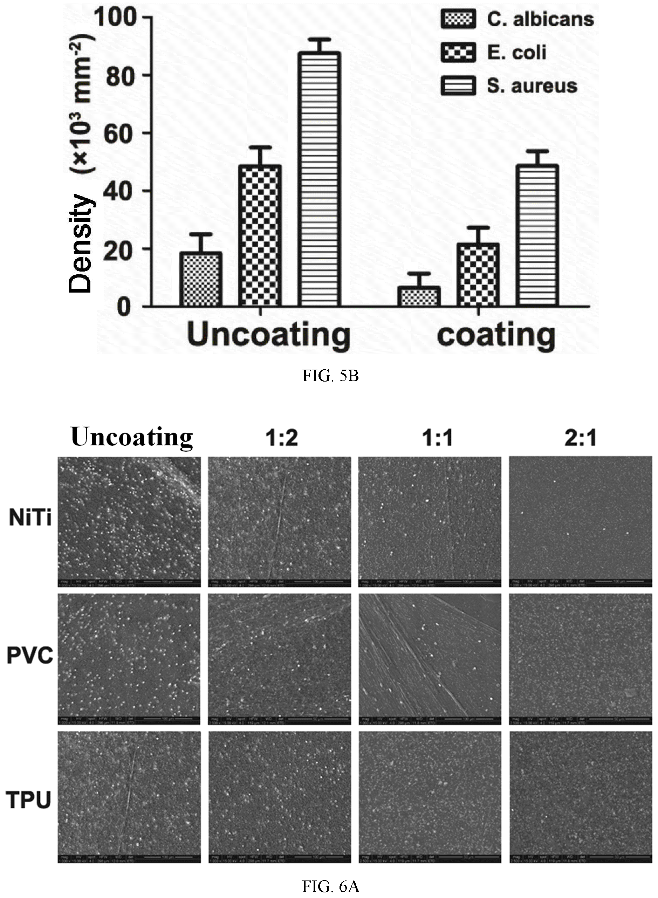

The antifouling radiopaque coating material of Example 1 was tested for resistance to bacterial adhesion. In terms of resistance to bacterial adhesion, the bacterial-adsorption resistance of the antifouling radiopaque coating was characterized and tested using *Candida albicans* (*C*. *albicans*), *Escherichia coli* (*E*. *coli*), and *Staphylococcus aureus* (*S*. *aureus*). The samples were immersed in three live bacteria suspensions at a high-concentration (10⁸/mL). The live bacteria were replaced every three days in the live bacteria suspensions. After two weeks of immersion, the samples were removed and air-dried, and the number of bacteria on the samples was observed using a scanning electron microscope (SEM). The solvent used was dialysate to maintain bacterial activity. The control groups were blank controls. The results are shown in FIGS. 5A and 5B.

As shown in FIG. 5A, after two weeks of exposure to the three different bacterial strains, large visible bacterial colonies were present on the control groups with no antifouling radiopaque coating, whereas no significant bacterial colony growth was observed on the samples coated with the antifouling radiopaque coating. Furthermore, the adhered bacterial colonies were quantitatively analyzed, and the results are shown in FIG. 5B. The samples coated with the antifouling radiopaque coating showed significantly reduced bacterial adhesion, with a 95% reduction in the amount of adhered bacteria after two weeks.

### Example 6

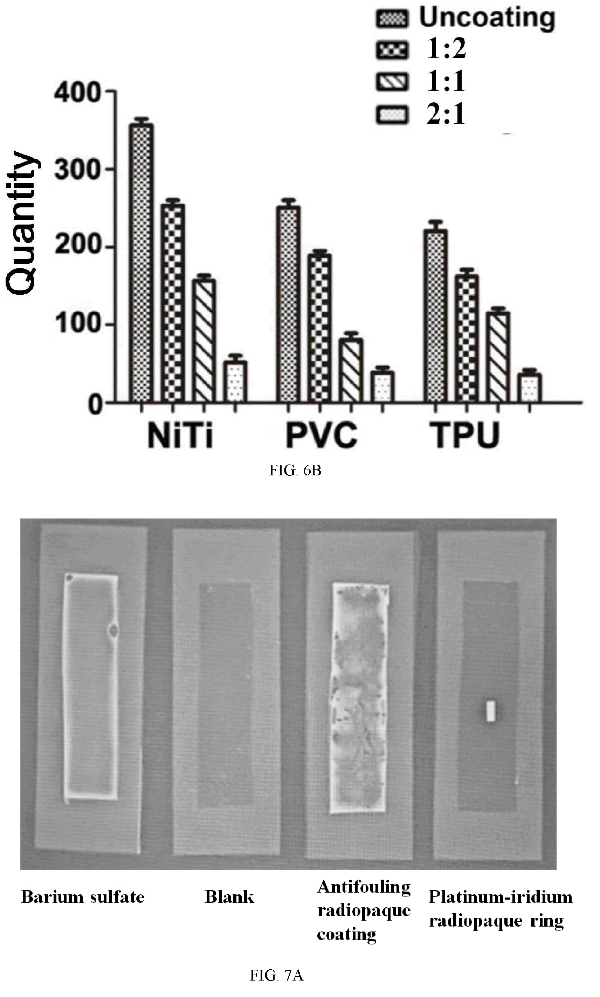

The antifouling radiopaque coating material of Example 1 was tested for platelet-adhesion resistance. The antifouling radiopaque coating was applied onto different substrate materials including NiTi, PVC, and TPU. Platelets were brought into contact with the test materials, washed, and observed under a scanning electron microscope (SEM) to study the effect of the antifouling radiopaque coating on platelet adhesion. The coating concentrations (solution concentrations) for the experimental groups were 100 mg/mL, 200 mg/mL, and 300 mg/mL, respectively, while the control groups were blank negative controls. The results are shown in FIGS. 6A and 6B. As shown in FIG. 4A, the antifouling radiopaque coating exhibited significant platelet-adhesion resistance on all three materials. Quantitative analysis of the quantity of adhered platelets was performed, and the results are shown in FIG. 6B. As can be seen, as the coating concentration increased, the platelet-adhesion resistance is significantly enhanced.

### Example 7

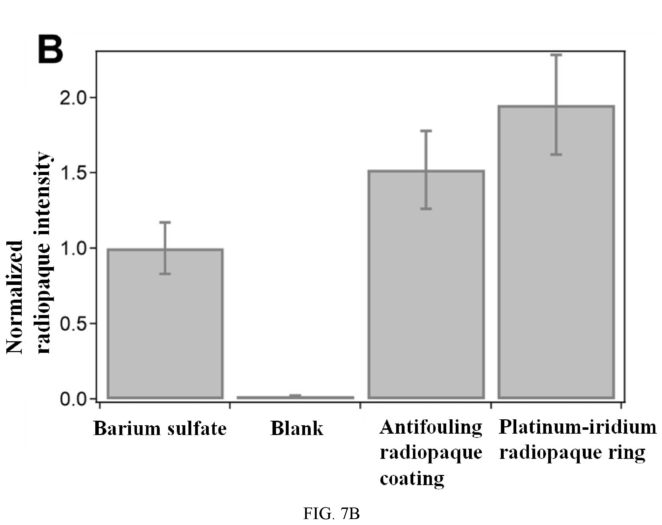

The antifouling radiopaque coating material of Example 1 was tested for X-ray imaging. A barium sulfate solution and the antifouling radiopaque coating material of the present application were each coated onto rectangular glass substrates at a coating concentration (solution concentration) of 100 mg/mL. A blank group and a commercial platinum-iridium radiopaque ring were used as controls. The results are shown in FIGS. 7A and 7B, where FIG. 7A shows X-ray imaging photographs of different radiopaque methods, and FIG. 7B is a graph showing the normalized radiopaque intensity analysis. As can be seen, the radiopaque performance of the antifouling radiopaque coating material of the present application was similar to the radiopaque performance of barium sulfate; although the radiopaque performance of the antifouling radiopaque coating material was slightly lower than the radiopaque performance of the commercial platinum-iridium radiopaque ring, it was far superior to the blank control group. Therefore, the antifouling radiopaque coating material of the present application exhibits an X-ray radiopaque performance comparable to that of barium sulfate.

From the above, it can be seen that the antifouling radiopaque coating material of the present application has excellent coating performance, antifouling performance, and X-ray radiopaque performances.

With the modified phosphorylcholine polymer serving as the antifouling unit and the bound barium serving as the radiopaque unit, the antifouling radiopaque coating material of the present application exhibits both excellent antifouling performance and radiopaque performance. The siloxane groups in the antifouling unit can form covalent bonding to the substrate surface to enable the coating to be firmly bound to the substrate, thereby ensuring the stability and safety of the antifouling radiopaque coating material of the present application on the substrate. For example, the siloxane groups can be covalently bound to silicon-based substrate materials, providing excellent stability without loss or detachment over time and significantly improving the durability and reliability of the radiopaque coating. The siloxane groups can also be bound to other substrate surfaces through hydrophobic interactions or van der Waals forces, thereby offering flexibility and applicability for the widespread use of the radiopaque coating. The antifouling radiopaque coating material of the present application can be applied to the surfaces of various substrates, including silicon-based substrates (for example, glass substrates), metal substrates, and various plastic and polymer substrates (such as silicone rubber, polyethylene, polypropylene, polyvinyl chloride, polystyrene or polyurethane), demonstrating broad applicability. The application fields of the antifouling radiopaque coating material are extensive, including, for example, medical devices, optical lenses, and industrial printing. The coating imparts both antifouling and X-ray imaging functions to the coated object, exhibiting excellent X-ray absorption performance during imaging, particularly providing accurate and clear radiopaque performances for medical imaging and offering reliable image information for medical radiography.

The applicant has stated that although the antifouling radiopaque coating material, the preparation method therefor and the use thereof of the present application are described through the preceding embodiments, the present application is not limited to the preceding embodiments, which means that the implementation of the present application does not necessarily depend on the preceding embodiments. It should be apparent to those skilled in the art that any improvements made to the present application and equivalent replacements of raw materials adopted, additions of adjuvant ingredients, selections of specific manners, etc. in the present application all fall within the scope of protection and disclosure of the present application.

## Claims

1. A novel antifouling radiopaque coating material, wherein the antifouling radiopaque coating material has a structure represented by Formula I: 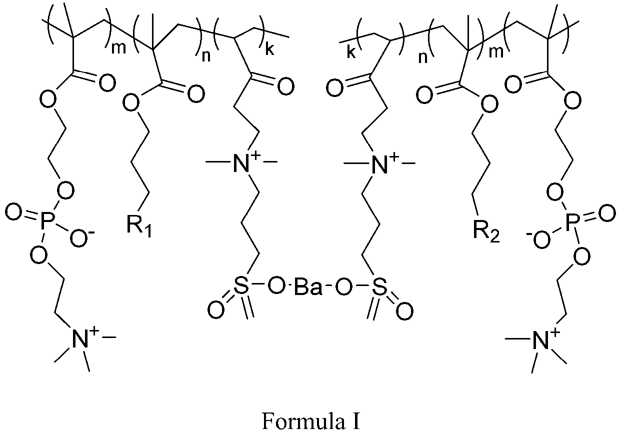 wherein m = 1 - 40, n = 1 - 40, k = 1 - 5, R₁ and R₂ each are a siloxane-containing group, and R₁ and R₂ are the same or different.

2. The novel antifouling radiopaque coating material according to claim 1, wherein m:n = 1:1 - 4:1.

3. The novel antifouling radiopaque coating material according to claim 1 or 2, wherein R₁ and R₂ are independently selected from tris(trimethylsiloxy)silane or trimethoxysilane, structures of which are as follows:

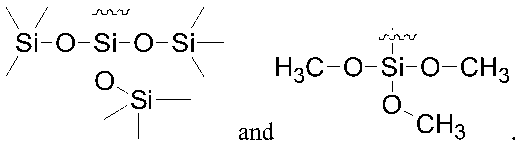

4. A preparation method for the novel antifouling radiopaque coating material according to any one of claims 1 to 3, comprising the following steps:
(1) preparing a phosphorylcholine polymer modified with siloxane and sulfonate groups; and
(2) adding a barium salt to a solution containing the phosphorylcholine polymer modified with siloxane and sulfonate groups, and reacting to obtain the novel antifouling radiopaque coating material.

5. The preparation method according to claim 4, wherein step (1) specifically comprises the following step: reacting 2-methacryloyloxyethyl phosphorylcholine with a siloxane compound and (2-(methacryloyloxy)ethyl)dimethyl-(3-sulfopropyl)ammonium hydroxide to obtain the phosphorylcholine polymer modified with siloxane and sulfonate groups.

6. The preparation method according to claim 5, wherein the siloxane compound is methacryloxypropyltris(trimethylsiloxy)silane, methacryloxypropyltris(trimethoxy)silane, or vinyltrimethoxysilane.

7. The preparation method according to claim 5 or 6, wherein a molar ratio of the 2-methacryloyloxyethyl phosphorylcholine to the siloxane compound is 1:1 - 8:1.

8. The preparation method according to any one of claims 5 to 7, wherein the reaction between the 2-methacryloyloxyethyl phosphorylcholine and the siloxane compound is carried out in the presence of 4-cyano-4-(thiobenzoylthio)pentanoic acid; and
optionally, a molar ratio of the 2-methacryloyloxyethyl phosphorylcholine to the 4-cyano-4-(thiobenzoylthio)pentanoic acid is 1:1 - 5:1.

9. The preparation method according to any one of claims 5 to 8, wherein the reaction between the 2-methacryloyloxyethyl phosphorylcholine and the siloxane compound is carried out in the presence of an initiator;
optionally, the initiator is selected from azobisisobutyronitrile; and
optionally, a molar ratio of the 2-methacryloyloxyethyl phosphorylcholine to the initiator is 1:1 - 5:1.

10. The preparation method according to any one of claims 5 to 9, wherein a molar ratio of the 2-methacryloyloxyethyl phosphorylcholine to the (2-(methacryloyloxy)ethyl)dimethyl-(3-sulfopropyl)ammonium hydroxide is 2:1 - 8:1.

11. The preparation method according to any one of claims 4 to 10, wherein the reaction in step (1) is carried out at a temperature of 60 °C - 70 °C for 16 - 64 h;
optionally, a solvent for the reaction in step (1) is one or a mixture of at least two of *n*-propanol, methanol, acetone, tetrahydrofuran, *n*-butanol, or isopropanol;
optionally, the reaction in step (1) is carried out under nitrogen protection;
optionally, step (1) comprises the following operations: dissolving 2-methacryloyloxyethyl phosphorylcholine, 4-cyano-4-(thiobenzoylthio)pentanoic acid, and azobisisobutyronitrile in *n*-propanol; and under nitrogen protection, heating to 60 °C - 70 °C, stirring and reacting for 16 - 32 h, adding a siloxane compound and (2-(methacryloyloxy)ethyl)dimethyl-(3-sulfopropyl)ammonium hydroxide, and continuing the reaction for another 16 - 32 h to obtain the phosphorylcholine polymer modified with siloxane and sulfonate groups; and
optionally, after the reaction in step (1) is completed, the preparation method further comprises a post-treatment step, wherein the post-treatment step comprises precipitating an obtained reaction solution in diethyl ether, filtering, and drying.

12. The preparation method according to any one of claims 4 to 11, wherein the barium salt in step (2) is selected from one or a mixture of at least two of barium chloride, barium sulfate, barium nitrate, or barium carbonate;
optionally, a molar ratio of the phosphorylcholine polymer modified with siloxane and sulfonate groups to an addition amount of the barium salt in step (2) ranges from 1:1 to 4:1, and optionally, is 1:1;
optionally, a solvent in the solution in step (2) is a mixed solvent of methanol and water; and
optionally, the reaction in step (2) is carried out at room temperature for 6 - 12 h.

13. Use of the novel antifouling radiopaque coating material according to any one of claims 1 to 3 in coating a substrate surface;
wherein optionally, the substrate surface comprises a silicon-based surface, a glass substrate surface, a metal substrate surface, or a polymer substrate surface.

14. An antifouling radiopaque coating, wherein a raw material for preparing the antifouling radiopaque coating comprises the novel antifouling radiopaque coating material according to any one of claims 1 to 3.

15. Use of the novel antifouling radiopaque coating material according to any one of claims 1 to 3 in a medical device, a medical material, an optical lens or industrial printing.
